# EUROPÄISCHE PATENTSCHRIFT

(11) **EP 0 771 787 B1**
(45) Veröffentlichungstag und Bekanntmachung des Hinweises auf die Patenterteilung: **09.06.1999**
(21) Anmeldenummer: 96116676.6
(22) Anmeldetag: 17.10.1996
(51) Int. Cl.: C07C 255/10, C07C 253/30

(54) **Verfahren zur Herstellung von 2-Chloracrylnitril**
Process for the preparation of 2-chloroacrylonitrile
Procédé de préparation de 2-chloroacrylonitrile

(30) Priorität: 30.10.1995 DE 19540358
(43) Veröffentlichungstag der Anmeldung: 07.05.1997
(73) Patentinhaber: BAYER AG, 51368 Leverkusen (DE)
(72) Erfinder: Mais, Franz-Josef, Dr., 40591 Düsseldorf (DE); Essert, Thomas, Dr., 51491 Overath (DE); Fiege, Helmut, Dr., 51373 Leverkusen (DE); Dürholz, Friedrich, Dr., 42897 Remscheid (DE); Steffan, Guido, Dr., 51519 Odenthal (DE)

(56) Entgegenhaltungen:
- EP-A- 0 030 869
- DE-B- 1 064 502
- DE-B- 1 076 673
- PATENT ABSTRACTS OF JAPAN vol. 6, no. 261 (C-141) [1139] , 21.Dezember 1982 & JP 57 154156 A (MITSUI TOATSU KAGAKU K.K.)
- DATABASE WPI Week 8135 Derwent Publications Ltd., London, GB; AN 81-63453d XP002023001 & JP 56 087 548 A (MITSUI TOATSU CHEM INC) , 16.Juli 1981

## Beschreibung

Die vorliegende Erfindung betrifft ein Verfahren zur Herstellung von 2-Chloracrylnitril durch Addition von elementarem Chlor an Acrylnitril und anschließende thermische Spaltung des gebildeten 2,3-Dichlorpropionitrils.

2-Chloracrylnitril ist ein wertvollen Zwischenprodukt, z.B. zur Herstellung von Pflanzenschutzmitteln (siehe z.B. US-PS 5 145 986).

Die Herstellung von 2,3-Dichlorpropionitril durch Addition von Chlor an Acrylnitril ist bekannt. Die US-PS 2 390 470 beschreibt die direkte Chlorierung von Acrylnitril mit Chlor unter Belichtung, wobei die Ausbeuten an 2,3-Dichlorpropionitril bestenfalls 80 % betragen. Die DE-OS 1 568 161 beansprucht diese Reaktion mit Zusätzen von beispielsweise Hydrogenphosphaten. Gute Ausbeuten von über 90 % erreicht man jedoch nur bei Reaktionstemperaturen von höchstens 0°C. Außerdem müssen die heterogenen anorganischen Bestandteile des Reaktionsgemisches vor der weiteren Verwendung durch Filtration entfernt werden, wobei ein belasteter anorganischer Abfall anfällt.

In der US-PS 2 429 031 wird die Chlorierung von Acrylnitril in Gegenwart katalytischer Mengen Chlorwasserstoff beschrieben. Die damalige Deutung der Versuchsergebnisse war jedoch fehlerhaft, denn in Gegenwart von freiem Halogenwasserstoff ist das Hauptprodukt 2,2,3-Trichlorpropionitril.

In Angew. Chem. 60, 311 bis 312 (1948) wird die Chlorierung von Acrylnitril in Gegenwart von Pyridin als Katalysator beschrieben. Die Weiterverarbeitung des erhaltenen 2,3-Dichlorpropionitrils zu 2-Chloracrylnitril ist ebenfalls beschrieben. Hierzu muß das 2,3-Dichlorpropionitril jedoch zunächst destilliert werden. Die dort angegebene Ausbeute von 95 % an 2,3-Dichlorpropionitril konnte bei einer Nacharbeitung (siehe J. Org. Chem 26, 2325 bis 2327 (1961)) nicht bestätigt werden. In der letztgenannten Literaturstelle ist auch die thermische Spaltung eines rohen, d.h. pyridinhaltigen 2,3-Dichlorpropionitrils zu 2-Chloracrylnitril beschrieben. Die Ausbeute an reinem 2-Chloracrylnitril beträgt jedoch nur unbefriedigende 60 %.

Die JP-OS 56-087 548 beansprucht die Chlorierung von Acrylnitril in Gegenwart von Säureamiden, z.B. Dimethylformamid, wobei destilliertes 2,3-Dichlorpropionitril in einer Ausbeute von 90 % erhalten wird.

In zahlreichen Patentanmeldungen (EP-OS 30 869, EP-OS 59 033, JP-OS 57-064 656, JP-OS 57-154 156, JP-OS 57-136 556 und JP-OS 01-258 653) ist die Chlorierung von Acrylnitril zu 2,3-Dichlorpropionitril in Gegenwart heterogener Katalysatoren beschrieben. Als Katalysatoren werden z.B. Alkalicarbonate, Alkalihydrogencarbonate, Alkalihydrogen- und -dihydrogenphosphate, Kombinationen aus gegebenenfalls substituiertem Pyridin mit Alkalicarbonaten und Kombinationen aus Kaliumcarbonat mit Polyvinylpyridin eingesetzt. Die Ausbeuten sind in der Regel gut, oft über 95 %, die Reinheiten liegen im Rohprodukt bei ca. 98 %. Der heterogene Katalysator muß vor der weiteren Umsetzung, z.B. der Spaltung in 2-Chloracrylnitril, durch Filtration entfernt werden, wobei ein hochbelasteter, schwer entsorgbarer fester Abfall anfällt. Anschließend wird das Rohprodukt destilliert, wobei in der Regel keine Spaltung zu 2-Chloracrylnitril eintritt (siehe z.B. EP-OS 30 869, Beispiel 2).

Die JP-OS 56-100 754 beschreibt die Chlorierung von Acrylnitril mit dem Ziel der direkten Zersetzung des rohen 2,3-Dichlorpropionitrils zu 2-Chloracrylnitril. Das Verfahren besitzt jedoch schwerwiegende Nachteile. Der anorganische heterogene "Katalysator" für die Chlorierung, im allgemeinen Alkali- oder Erdalkali-hydrogen- oder -dihydrogenphosphat, muß in einer Menge von 120 mol-%, bezogen auf Acrylnitril, eingesetzt werden. Außerdem ist die Reaktionstemperatur in der Nähe von 0°C zu halten, d.h. die Abführung der Reaktionswärme der stark exothermen Chloraddition ist aufwendig. Die Ausbeute an 2-Chloracrylnitril (nach Chlorierung, thermischer Spaltung und Destillation) beträgt nur 80,2 % (70,2 g 2-Chloracrylnitril aus 53 g Acrylnitril). Der größte Nachteil dieses Verfahrens ist jedoch die Durchführung sämtlicher Verfahrensschritte in Gegenwart der übergroßen Menge an Phosphaten. Während der thermischen Spaltung und vor allem bei der abschließenden Destillation führt das im Sumpf der Reaktions- und Destillationsapparatur zu heftigen Stößen. Zum Abdestillieren des Produkts sollte der phosphathaltige Rückstand bis zur Trockene eingedampft werden, was in technischem Maßstab praktisch nicht durchführbar ist.

Außer in der bereits erwähnten Literatur ist die Abspaltung von Chlorwasserstoff aus 2,3-Dichlorpropionitril auch noch aus einigen anderen Veröffentlichungen bekannt. Es werden die unterschiedlichsten Reagenzien für die Abspaltung von Chlorwasserstoff beschrieben, so in der US-PS 2 385 550 sekundäre und tertiäre Amine (85 % Ausbeute nach Destillation), in der DE-AS 883 891 Salze von Polycarbonsäuren im wäßrigen Medium (ein Beispiel für die Spaltung von 2,3-Dichlorpropionitril ist nicht vorhanden), in der US-PS 2 862 963 saure Reagenzien wie aromatische Sulfonsäuren und polymere sulfonsaure Salze (das die Spaltung von 2,3-Dichlorpropionitril betreffende Beispiel 4 enthält keine Angabe der Ausbeute), in der US-PS 2 870 192 konzentrierte Schwefelsäure (siehe Beispiel 2, allerdings ohne Angabe der Ausbeute), in der US-PS 3 361 786 Kaliumfluorid (Ausbeute maximal 83,5 %, siehe Tabelle 3) und in der DE-OS 1 768 807 und US-PS 3 845 095 wäßrige Lösungen von Alkalihydrogen- und -dihydrogenphosphaten. Das letztgenannte, sehr aufwendige Verfahren liefert bestenfalls 87,5 % Ausbeute (Beispiel 1 der DE-OS 1 768 807) an einem Material, das durch Phasentrennung von einer wäßrigen Phase stammt. In dieser feuchten Form ist das 2-Chloracrylnitril jedoch technisch unbrauchbar, so daß noch eine aufwendige und ausbeutemindernde Trocknung erfolgen muß. Die GB-PS 1 287 854 beschreibt die Abspaltung von Chlorwasserstoff mit wäßrigem Ammoniak, wobei die Ausbeute laut Beispiel 2 80 % beträgt.

Die DE-AS 1 150 381 beansprucht ein Verfahren zur thermischen Spaltung von 2,3-Dichlorpropionitril in Gegenwart von Eisen, Aluminium oder deren Chloriden als Katalysatoren. Die Ausbeute des an sich günstigen Verfahrens ist mit 95 % gut. Es muß aber reines, zuvor zwischenaufgearbeitetes 2,3-Dichlorpropionitril, in dem die Chlorierkatalysatoren nicht mehr enthalten sind, eingesetzt werden.

Schließlich beschreibt die US-PS 2 231 363 ein direktes Gasphasenverfahren, bei dem aus Acrylnitril und Chlor bei 200 bis 500°C in einem Schritt unter in situ Erzeugung und Spaltung von 2,3-Dichlorpropionitril 2-Chloracrylnitril erhalten werden kann. Die Ausbeute beträgt jedoch nur 40 %.

An dem genannten Stand der Technik ist ausgesprochen nachteilig, daß bei vielen Verfahren nach der Chlorierung von Acrylnitril zu 2,3-Dichlorpropionitril eine Entfernung der Katalysatoren (mit gegebenenfalls großen Entsorgungsproblemen) und eine weitere Reinigung des 2,3-Dichlorpropionitrils durch Destillation nötig ist. Das reine 2,3-Dichlorpropionitril kann dann in die Spaltung zu 2-Chloracrylnitril eingesetzt werden. Die wenigen Veröffentlichungen, die einen Einsatz von rohen Katalysatoren oder sonstige Reagenzien enthaltendem 2,3-Dichlorpropionitril beschreiben, laufen mit unbefriedigend niedriger Ausbeute ab und sind zum Teil in technischem Maßstab kaum durchführbar.

Es besteht deshalb noch immer ein Bedürfnis nach einem einfachen Verfahren zur Herstellung von 2-Chloracrylnitril in hohen Ausbeuten, das auch in technischem Maßstab gut durchführbar ist.

Es wurde nun ein Verfahren zur Herstellung von 2-Chloracrylnitril durch Chlorierung von Acrylnitril und anschließender thermischer Spaltung des gebildeten 2,3-Dichlorpropionitrils gefunden, das dadurch gekennzeichnet ist, daß man Acrylnitril in Gegenwart eines Katalysatorsystems, das Dimethylformamid und Pyridin und/oder Pyridinderivate enthält, chloriert und das dabei erhaltene rohe 2,3-Dichlorpropionitril in Gegenwart desselben Katalysatorsystems ohne Zusatz weiterer Katalysatoren thermisch spaltet.

In das erfindungsgemäße Verfahren kann in die erste Stufe (Chlorierung) üblicherweise im Handel erhältliches Acrylnitril eingesetzt werden. Solches Acrylnitril enthält häufig Polymerisationinhibitoren, z.B. Hydrochinon, Hydrochinonmonomethylether, Phenothiazin, tert.-butylierte Phenole und/oder Kresole in Mengen von z.B. 40 bis 100 ppm. Die Gegenwart von solchen Mengen Polymerisationsinhibitoren stört das erfindungsgemäße Verfahren nicht. Es ist auch möglich Acrylnitril einzusetzen, das keine Polymerisationsinhibitoren enthält.

Die erfindungsgemäße erste Verfahrensstufe der Chlorierung von Acrylnitril kann in flüssiger Phase durchgeführt werden, wobei man mit oder ohne Zusatz eines Lösungsmittels arbeiten kann. Geeignete Lösungsmittel sind solche, die durch Chlor unter den Reaktionsbedingungen nicht angegriffen werden, z.B. Tetrachlorkohlenstoff, Perchlorethylen und andere chlorierte Kohlenwasserstoffe. Bevorzugt wird kein Lösungsmittel zugesetzt.

Als Chlorierungsmittel kann gasförmiges Chlor verwendet werden. Die Menge des Chlorierungsmittels kann z.B. im Bereich von 0,9 bis 1,1 mol pro mol eingesetztem Acrylnitril liegen. Bevorzugt beträgt diese Menge 0,95 bis 1,05 mol, insbesondere 0,99 bis 1,05 mol.

Ein wesentliches Merkmal des erfindungsgemäßen Verfahrens ist der Einsatz eines Katalysatorsystems aus Dimethylformamid (DMF) und Pyridin und/oder einem oder mehreren Pyridinderivaten. Dimethylformamid kann z.B. in einer Menge von 0,1 bis 20 mol-%, bezogen auf Acrylnitril, eingesetzt werden. Bevorzugt liegt diese Menge bei 0,5 bis 10 mol-%, besonders bevorzugt bei 1 bis 7,5 mol-%.

Die Pyridinkomponente kann z.B. in einer Menge von 0,05 bis 10 mol-%, bezogen auf Acrylnitril, eingesetzt werden. Bevorzugt beträgt diese Menge 0,1 bis 7,5 mol-%, besonders bevorzugt 0,5 bis 5 mol-%.

Pyridin und Pyridinderivate können jeweils für sich alleine oder in beliebigen Gemischen untereinander eingesetzt werden. Gemeinsam werden Pyridin und Pyridinderivate im folgenden auch als Pyridinkomponente bezeichnet.

Die Pyridinkomponente kann z.B. Verbindungen der Formel (I) enthalten 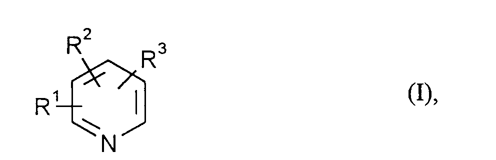 in der
- R¹, R² und R³: unabhängig voneinander jeweils Wasserstoff, geradkettiges, verzweigtes oder cyclisches C₁-C₆-Alkyl, Phenyl oder Benzyl bedeuten und wobei zwei dieser Reste R¹, R² und R³, wenn sie benachbart sind, gemeinsam auch eine -(CH₂)₃- oder -(CH₂)₄-Gruppe bedeuten können.

Geradkettiges, verzweigtes oder cyclisches C₁-C₆-Alkyl ist beispielsweise Methyl, Ethyl, Propyl, Isopropyl, Butyl, tert.-Butyl und Cyclohexyl. Bevorzugt bedeuten R¹, R² und R³ unabhängig voneinander jeweils Wasserstoff, geradkettiges oder verzweigtes C₁-C₄-Alkyl, Phenyl oder Benzyl, besonders bevorzugt Wasserstoff, Methyl oder Ethyl.

Das Molverhältnis von Dimethylformamid zur Pyridinkomponente kann z.B. in einem Bereich von 20:1 bis 0,5:1 liegen. Bevorzugt beträgt dieses Verhältnis 10:1 bis 1:1, insbesondere 7,5:1 bis 2:1.

Die Reaktionstemperatur für die Chlorierung kann z.B. 10 bis 60°C betragen. Bevorzugt liegt sie bei 20 bis 50°C, insbesondere bei 25 bis 45°C.

Die Chlorierung kann bei Normaldruck, vermindertem oder erhöhtem Druck durchgeführt werden. Bevorzugt ist Normaldruck oder ein apparatebedingter leichter Überdruck.

Erfindungsgemäß wird zur Durchführung der thermischen Spaltung des rohen 2,3-Dichlorpropionitrils kein weiterer Katalysator zugegeben.

Die erfindungsgemäße zweite Verfahrensstufe (thermische Spaltung) kann ebenfalls in flüssiger Phase durchgeführt werden. Hierbei kann sowohl in Verdünnung mit einem, vorzugsweise hochsiedenden Lösungsmittel, aber auch ohne Lösungsmittelzusatz gearbeitet werden. Geeignete hochsiedende Lösungsmittel, die durch Chlorwasserstoff, Acrylnitril oder Chlorierungsprodukte davon unter den Reaktionsbedingungen nicht angegriffen werden, sind beispielsweise Polychlorbenzole wie 1,2-Dichlorbenzol und 1,2,4-Trichlorbenzol, hochsiedende Mineralöle, Polyethylenglykole mit mittleren Molekulargewichten von beispielsweise 300 bis 400, Dimethylformamid und N-Methylpyrrolidon.

Bevorzugt wird die thermische Spaltung ohne Zusatz eines Lösungsmittels durchgeführt.

Zur Vermeidung der Polymerisation des gebildeten 2-Chloracrylnitrils unter den Bedingungen der thermischen Spaltung kann dem Spaltgemisch ein üblicher Polymerisationsinhibitor zugefügt werden. Als Beispiele seien Hydrochinon, Hydrochinonmonomethylether und Phenothiazin genannt.

In die Spaltung kann man direkt das aus der Chlorierung von Acrylnitril kommende 2,3-Dichlorpropionitril enthaltende Reaktionsgemisch einsetzen. Bei der thermischen Spaltung destilliert das gebildete 2-Chloracrylnitril und der gebildete Chlorwasserstoff ab. Die Spaltreaktion kann z.B. bei 80 bis 160°C, bevorzugt bei 90 bis 140°C und besonders bevorzugt bei 90 bis 110°C vorgenommen werden (jeweils gemessen im Sumpf). Die thermische Spaltung kann bei Normaldruck, erniedrigtem oder erhöhtem Druck durchgeführt werden. Bevorzugt ist Normaldruck oder ein apparatebedingter leichter Überdruck.

Die Ausführung des erfindungsgemäßen Verfahrens kann in verschiedenen Varianten erfolgen. Als Beispiel seien die folgenden genannt: Die erste Stufe des Verfahrens, die Chlorierung von Acrylnitril, läßt sich sowohl kontinuierlich als auch diskontinuierlich durchführen. Eine beispielhafte Ausführungsform ist die folgende: In einem Chloriergefäß legt man Acrylnitril vor, gibt die beiden Katalysatorkomponenten zu und leitet Chlor bei der gewünschten Reaktionstemperatur ein. Nach kurzem Nachrühren kann das Material entweder direkt zur Spaltung eingesetzt oder zwischengelagert werden. Die zweite Stufe, die Spaltung des katalysatorhaltigen 2,3-Dichlorpropionitrils, kann ebenfalls in verschiedenen Varianten ausgeführt werden. Beispielhafte Ausführungsformen sind z.B. die folgenden: In einem Gefäß wird die Gesamtmenge des 2,3-Dichlorpropionitrils vorgelegt und bis zur einsetzenden Spaltung erhitzt. Die entstehenden Produkte 2-Chloracrylnitril und Chlorwasserstoff werden über einen kurzen Kühler geleitet. Das im wesentlichen aus 2-Chloracrylnitril bestehende Kondensat kann gegebenenfalls zur Feinreinigung nochmals destilliert werden.

Eine weitere Ausführungsform ist wie folgt: In einer Destillationsblase wird eine Teilmenge des rohen 2,3-Dichlorpropionitrils bis zur einsetzenden Spaltung erhitzt. Das gebildete 2-Chloracrylnitril und der Chlorwasserstoff destillieren, gegebenenfalls über eine aufgesetzte Trennkolonne, ab. In gleichem Maße wird in den Sumpf das restliche rohe 2,3-Dichlorpropionitril zudosiert. Am Ende erfolgt noch eine kurze Nacherhitzung bis die Spaltung nachläßt. Besonders bei Verwendung einer Trennkolonne ist das erhaltene 2-Chloracrylnitril schon so rein, daß es ohne weitere Behandlung den bekannten Verwendungen zugeführt werden kann. Insbesondere ist eine zusätzliche Destillation dann nicht mehr nötig.

Bei dem erfindungsgemäßen Verfahren ist ausgesprochen überraschend und war bei dem bekannten Stand der Technik nicht zu erwarten, daß eine Kombination von Dimethylformamid mit Pyridinkomponenten für beide Stufen eine katalytische Wirkung besitzt und eine hohe Ausbeute sowohl bei der Chlorierung, als auch bei der thermischen Spaltung realisiert werden kann. Die nicht mehr nötige Zwischenreinigung des 2,3-Dichlorpropionitrils und die nicht mehr benötigten anorganischen, abzufiltrierenden und hochbelastenden Reagenzien sind große technische Fortschritte. Die Ausbeuten an 2-Chloracrylnitril sind hoch und liegen über beide Reaktionsstufen betrachtet im allgemeinen bei 85 bis 95 %.

Die folgenden Beispiele erläutern das erfindungsgemäße Verfahren ohne es auf die dort geschilderten Ausführungsformen einzuschränken.

### Beispiele

### Beispiele 1 bis 6

In einem lichtgeschützten Chlorierbecher wurden 2650 g Acrylnitril vorgelegt und mit dem Chlorierkatalysator versetzt. Unter Rühren wurde Chlor gasförmig unter die Flüssigkeitsoberfläche eindosiert. Es setzte eine exotherme Reaktion ein. Bei der gewünschten Reaktionstemperatur wurde eine weitere Temperaturerhöhung durch Einschalten einer effektiven Kühlung verhindert.

Insgesamt wurden 3550 g Chlor im Verlauf von 10 Stunden eingeleitet. Anschließend wurde noch 1 Stunde bei Reaktionstemperatur nachgerührt.

Die erhaltenen Reaktionsgemische konnten direkt in die Spaltung zu 2-Chloracrylnitril und Chlorwasserstoff eingespeist werden. Die Einzelheiten und Ergebnisse der Beispiele 1 bis 6 sind aus der Tabelle 1 ersichtlich.

**Tabelle 1**

| Bsp. | Katalysator | Reaktionstemperatur | Auswaage | 2,3-Dichlorpropionitril | |
|---|---|---|---|---|---|
| | | | | GC-Gehalt | Ausbeute |
| 1 | 3,5 mol-% DMF, 5 mol-% Pyridin | 30°C | 6515 g | 93,0 % | 97,7 % |
| 2 | 1 mol-% DMF, 5 mol-% Pyridin | 30°C | 6405 g | 94,2 % | 97,3 % |
| 3 | 1 mol-% DMF, 7 mol-% 2-Methylpyridin | 40°C | 6560 g | 92,8 % | 98,2 % |
| 4 | 3,5 mol-% DMF, 4 mol-% 2-Phenylpyridin | 30°C | 6640 g | 90,2 % | 96,6 % |
| 5 | 1 mol-% DMF, 3,5 mol-% 2-Methylpyridin | 35°C | 6380 g | 95,0 % | 97,7 % |
| 6 | 1 mol-% DMF, 3,5 mol-% 2,4-Dimethylpyridin | 35°C | 6415 g | 94,9 % | 98,2 % |

### Beispiel 7

In einem 2000 ml Mehrhalskolben mit aufgesetzter verspiegelter 1 m Vigreux-Kolonne wurden 1312 g (10,0 mol) des Produktes aus Beispiel 3 vorgelegt und mit 10 g Hydrochinon versetzt. Unter Rühren wurde erhitzt bis bei ca. 100°C die Spaltung einsetzte und dann bei 125 bis 130°C weitergerührt. Dabei destillierte 2-Chloracrylnitril und Chlorwasserstoff über die Kolonne ab. Am Kopf der Kolonne wurde über einen Kühler der Chlorwasserstoff abgeführt Das Kondensat wurde bei einem Rücklaufverhältnis von 1:1 warm in die Vorlage abgenommen. Die Kopftemperatur betrug 85°C. Die Ausbeute an 99 %igem 2-Chloracrylnitril (GC) war 760 g. Das entspricht 86,9 % über beide Reaktionsstufen.

### Beispiel 8

In einem 500 ml Mehrhalskolben mit aufgesetzter verspiegelter 1 m Vigreux-Kolonne wurden 131 g (1,0 mol) des Produktes aus Beispiel 3 vorgelegt und mit 5 g Hydrochinon versetzt. Man erhitzte unter Rühren auf 125 bis 130°C. 2-Chloracrylnitril und Chlorwasserstoff destillierten über die Kolonne ab. Der Chlorwasserstoff wurde wie in Beispiel 7 über einen Kühler am Kolonnenkopf abgeführt. Das 2-Chloracrylnitril-Destillat wurde bei einem Rücklaufverhältnis von 1:1 warm abgenommen. Die Kopftemperatur betrug 85°C. In dem Maße, in dem durch Spaltung 2,3-Dichlorpropionitril aus dem Sumpf verbraucht wurde, wurden im Verlaufe von 10 Stunden 1181 g (9,0 mol) des Produktes aus Beispiel 3 nachdosiert. Nach einer Nachheizphase von 30 Minuten bei 130 bis 135°C erhielt man insgesamt 810 g 2-Chloracrylnitril von ca. 99 %igem Gehalt. Das entspricht einer Ausbeute von 92,6 % über beide Reaktionsstufen.

### Beispiel 9

Das Verfahren des Beispiels 7 wurde mit 1281 g (10,0 mol) des Produktes aus Beispiel 2 wiederholt. Man erhielt 745 g (= 85,1 % über beide Reaktionsstufen) an 98,5 %igem 2-Chloracrylnitril.

## Patentansprüche

1. Verfahren zur Herstellung von 2-Chloracrylnitril durch Chlorierung von Acrylnitril und anschließende thermische Spaltung des gebildeten 2,3-Dichlorpropionitrils, dadurch gekennzeichnet, daß man Acrylnitril in Gegenwart eines Katalysatorsystems, das Dimethylformamid und Pyridin und/oder Pyridinderivate enthält, chloriert und das dabei erhaltene rohe 2,3-Dichlorpropionitril in Gegenwart desselben Katalysatorsystems ohne Zusatz weiterer Katalysatoren thermisch spaltet.

2. Verfahren nach Anspruch 1, dadurch gekennzeichnet, daß man Dimethylformamid in einer Menge von 0,1 bis 20 mol-%, bezogen auf Acrylnitril, einsetzt.

3. Verfahren nach Ansprüchen 1 und 2, dadurch gekennzeichnet, daß man Pyridin und/oder Pyridinderivate in einer Menge von 0,05 bis 10 mol-%, bezogen auf Acrylnitril, einsetzt.

4. Verfahren nach Ansprüchen 1 bis 3, dadurch gekennzeichnet, daß man Pyridin und/oder eines oder mehrere Pyridinderivate der Formel (I) einsetzt 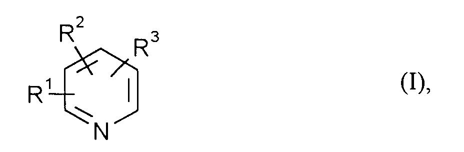 in der
R¹, R² und R³ unabhängig voneinander jeweils Wasserstoff, geradkettiges, verzweigtes oder cyclisches C₁-C₆-Alkyl, Phenyl oder Benzyl bedeuten und wobei zwei dieser Reste R¹, R² und R³, wenn sie benachbart sind, gemeinsam auch eine -(CH₂)₃- oder -(CH₂)₄-Gruppe bedeuten können.

5. Verfahren nach Anspruch 4, dadurch gekennzeichnet, daß man Pyridin und/oder eines oder mehrere Pyridinderivate der Formel (I) einsetzt, wobei R¹, R² und R³ unabhängig voneinander jeweils Wasserstoff, geradkettiges oder verzweigtes C₁-C₄-Alkyl, Benzyl oder Phenyl bedeuten.

6. Verfahren nach Ansprüchen 1 bis 5, dadurch gekennzeichnet, daß man die Chlorierung von Acrylnitril bei 10 bis 60°C durchführt.

7. Verfahren nach Ansprüchen 1 bis 7, dadurch gekennzeichnet, daß man die Chlorierung von Acrylnitril mit 0,9 bis 1,1 mol Chlor pro mol Acrylnitril durchführt,.

8. Verfahren nach Ansprüchen 1 bis 7, dadurch gekennzeichnet, daß man die thermische Spaltung des rohen 2,3-Dichlorpropionitrils bei einer Temperatur von 80 bis 160°C durchführt.

9. Verfahren nach Ansprüchen 1 bis 8, dadurch gekennzeichnet, daß das Molverhältnis von Dimethylformamid zu Pyridin und/oder Pyridinderivaten im Bereich 20:1 bis 0,5:1 liegt.

10. Verfahren nach Ansprüchen 1 bis 9, dadurch gekennzeichnet, daß man die Chlorierung bei 20 bis 50°C und die Spaltung bei 90 bis 140°C durchführt.

## Claims

1. Process for the preparation of 2-chloroacrylonitrile by chlorinating acrylonitrile and then thermally cleaving the 2,3-dichloropropionitrile formed, characterized in that acrylonitrile is chlorinated in the presence of a catalyst system comprising dimethylformamide and pyridine and/or pyridine derivatives and the resulting crude 2,3-dichloropropionitrile is subjected to thermal cleavage in the presence of the same catalyst system without the addition of further catalysts.

2. Process according to Claim 1, characterized in that dimethylformamide is employed in an amount of from 0.1 to 20 mol%, based on acrylonitrile.

3. Process according to Claims 1 and 2, characterized in that pyridine and pyridine derivatives is or are employed in an amount of from 0.05 to 10 mol%, based on acrylonitrile.

4. Process according to Claims 1 to 3, characterized in that pyridine and/or one or more pyridine derivatives of the formula (I) is or are employed 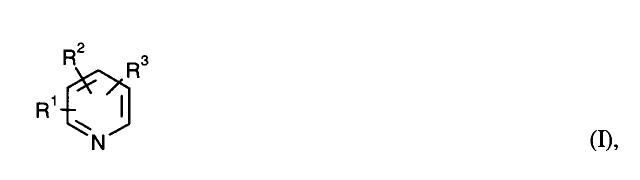 in which
R¹, R² and R³ independently of one another are each hydrogen, straight-chain, branched or cyclic C₁-C₆-alkyl, phenyl or benzyl and two of these radicals R¹, R² and R³, if adjacent, can also together be a - (CH₂)₃- or -(CH₂)₄-group.

5. Process according to Claim 4, characterized in that pyridine and/or one or more pyridine derivatives of the formula (I) is or are employed in which R¹, R² and R³ independently of one another are each hydrogen, straight-chain or branched C₁-C₄-alkyl, benzyl or phenyl.

6. Process according to Claims 1 to 5, characterized in that the chlorination of acrylonitrile is carried out at from 10 to 60°C.

7. Process according to Claims 1 to 6, characterized in that the chlorination of acrylonitrile is carried out with from 0.9 to 1.1 mol of chlorine per mole of acrylonitrile.

8. Process according to Claims 1 to 7, characterized in that the thermal cleavage of the crude 2,3-dichloropropionitrile is carried out at a temperature of from 80 to 160°C.

9. Process according to Claims 1 to 8, characterized in that the molar ratio of dimethylformamide to pyridine and/or pyridine derivatives is in the range from 20:1 to 0.5:1.

10. Process according to Claims 1 to 9, characterized in that the chlorination is carried out at from 20 to 50°C and the cleavage at from 90 to 140°C.

## Revendications

1. Procédé pour la préparation du 2-chloroacrylonitrile par chloration d'acrylonitrile et par scission thermique ultérieure du 2,3-dichloropropionitrile obtenu, caractérisé en ce qu'on soumet de l'acrylonitrile à une chloration en présence d'un système de catalyseur qui contient du diméthylformamide et de la pyridine et/ou des dérivés de la pyridine, et on soumet le 2,3-dichloropropionitrile brut obtenu en l'occurrence à une scission par voie thermique en présence du même système de catalyseur sans ajouter d'autres catalyseurs.

2. Procédé selon la revendication 1, caractérisé en ce qu'on met en oeuvre du diméthylformamide en une quantité de 0,1 à 20 moles % rapportés à l'acrylonitrile.

3. Procédé selon les revendications 1 et 2, caractérisé en ce qu'on met en oeuvre de la pyridine et/ou des dérivés de la pyridine en une quantité de 0,05 à 10 moles % rapportés à l'acrylonitrile.

4. Procédé selon les revendications 1 à 3, caractérisé en ce qu'on met en oeuvre de la pyridine et/ou un ou plusieurs dérivés de la pyridine répondant à la formule (I) 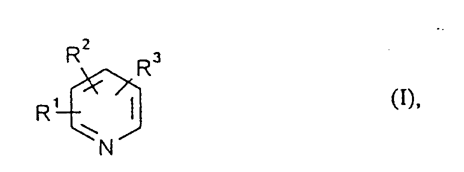 dans laquelle
R¹, R² et R³ représentent, indépendamment l'un de l'autre, respectivement un atome d'hydrogène, un groupe alkyle en C₁-C₆ à chaîne droite, à chaîne ramifiée ou cyclique, un groupe phényle ou un groupe benzyle et dans lequel deux de ces radicaux R¹, R² et R³, lorsqu'ils sont voisins, peuvent également former ensemble un groupe -(CH₂)₃- ou un groupe -(CH₂)₄-.

5. Procédé selon la revendication 4, caractérisé en ce qu'on met en oeuvre de la pyridine et/ou un ou plusieurs dérivés de la pyridine répondant à la formule (I) dans laquelle R¹, R² et R³ représentent, indépendamment l'un de l'autre, respectivement un atome d'hydrogène, un groupe alkyle en C₁-C₄ à chaîne droite ou à chaîne ramifiée, un groupe benzyle ou un groupe phényle.

6. Procédé selon les revendications 1 à 5, caractérisé en ce qu'on effectue la chloration de l'acrylonitrile à une température de 10 à 60°C.

7. Procédé selon les revendications 1 à 6, caractérisé en ce qu'on effectue la chloration de l'acrylonitrile avec de 0,9 à 1,1 mole de chlore par mole d'acrylonitrile.

8. Procédé selon les revendications 1 à 7, caractérisé en ce qu'on effectue la scission par voie thermique du 2,3-dichloropropionitrile brut à une température de 80 à 160°C.

9. Procédé selon les revendications 1 à 8, caractérisé en ce que le rapport molaire du diméthylformamide à la pyridine et/ou aux dérivés de la pyridine se situe dans le domaine de 20:1 à 0,5:1.

10. Procédé selon Des revendications 1 à 9, caractérisé en ce qu'on effectue la chloration à une température de 20 à 50°C et la scission à une température de 90 à 140°C.
